# EUROPEAN PATENT SPECIFICATION

(11) **EP 1 785 724 B1**
(45) Date of publication and mention of the grant of the patent: **08.08.2012**
(21) Application number: 05780986.5
(22) Date of filing: 17.08.2005
(51) Int. Cl.: G01N 27/447

(54) **METHOD OF LABELING WITH USE OF RARE EARTH FLUORESCENT COMPLEX AND RELEVANT METHOD OF ANALYSIS AND DETECTION**
MARKIERUNGSVERFAHREN UNTER VERWENDUNG EINES SELTENE-ERDEN-FLUORESZENZKOMPLEXES SOWIE DAMIT VERBUNDENES ANALYSE- UND NACHWEISVERFAHREN
PROCEDE DE MARQUAGE METTANT EN OEUVRE UN COMPLEXE FLUORESCENT DE TERRES RARES ET PROCEDE ASSOCIE D'ANALYSE ET DE DETECTION

(30) Priority: 17.08.2004 JP 2004237767
(43) Date of publication of application: 16.05.2007
(73) Proprietor: Japan Science and Technology Agency, Kawaguchi-shi, Saitama 332-0012 (JP)
(72) Inventor: MATSUMOTO, Kazuko, Setagaya-ku, Tokyo 155-0032 (JP); YAMAGUCHI, Yoshinori, Higashimurayama-shi, Tokyo 189-0021 (JP); HASHINO, Kimikazu, Takatsuki-shi, Osaka 569-0082 (JP)
(74) Representative: Gordon, Kirsteen Helen
(86) International application number: PCT/JP2005/015307
(87) International publication number: WO 2006/019179

(56) References cited:
- WO-A1-96/38724
- WO-A1-02/082083
- WO-A1-03/076938
- WO-A1-03/076938
- WO-A1-03/083472
- WO-A2-03/075772
- US-A- 5 637 509
- US-B1- 6 540 897
- HOSOYA C.: 'Jisedai DNA Microarray System no Kaihatsu: Shinki Kidorui Keiko Sakutai DTBTA-Eu3+ o Mochiita Kakusan Kenshutsu' THE MOLECULAR BIOLOGY SOCIETY OF JAPAN NENKAI PROGRAM KOEN YOSHISHU vol. 26, 25 November 2003, page 653

## Description

### Technical Field

The present invention relates to the use of a rare earth fluorescent complex DTBTA-E_{U}³⁺ to label a sample in a method of detecting and analysing said sample by electrophoresis.

### Background Art

From the past, in order to clarify a function of a living organism by a cell level, protein, peptide, nucleic acid, amino acid, carbohydrate, and neurotransmission related substance in a living body have been analyzed. A high performance liquid chromatography equipped with a mass spectrometer has been used as the analyzing means, but the number of theoretical plates thereof is inferior to capillary electrophoresis. It has been reported that the number of theoretical plates in a separation analysis of oligonucleotide is 1,000,000 with capillary electrophoresis and 30,000 with high performance liquid chromatography (for example, refer to Non-Patent Document 1). In addition, since absolute amount of a sample required for the analysis is smaller than high performance liquid chromatography (by several ml for high performance liquid chromatography and pl level for a capillary electrophoresis method), the capillary electrophoresis is used for the separation analysis. Further, capillary electrophoresis has been well known for its high resolution performance as it is being used for Human Genome Project and is frequently used for the analysis of protein, amino acid, and the like in these days.

However, in many cases, since the detection is performed by measuring a absorbance of the sample, there is a weak point that the detection limit is often deteriorated and differs in accordance with the types of the sample. In order to solve such a weak point, there is a method of measuring amino acid by labeling amino acid with a fluorescent material (for example, refer to Non-Patent Document 2), but the method of labeling is hardly employed to an actual measurement because the method is complicated and high-power laser is used for the detection. As other method of measuring amino acid, for example, a method of measuring by using an absorption of amino acid derived to strengthen the absorption in an UV portion, specifically phenylthiohydantoin-amino acid using phenylisothiocyanate as a derivatization reagent and dansyl-amino acid using dansylchloride as the derivatization reagent, has been widely used (for example, refer to Non-Patent Document 3). However, the detection limit is in mM levels.

The analysis of peptide or protein is also carried out by using capillary electrophoresis. In the analysis of peptide, protein is degraded by enzymes in many cases and great efforts are given to improve the separation of peptide. In addition, only the absorption method is used for the detection. For the separation analysis of protein, the detection is performed by the absorption method. However, for detecting the absorption of example, when capillary gel electrophoresis filled with polymer is used, the detection limit may be deteriorated by the absorption of gel or polymer filled thereto. In addition, a method of analyzing protein by measuring fluorescence using excimer laser has been used in these days (for example, refer to Non-Patent Document 4). In such a case, protein to be analyzed is labeled with 3-(2-furoyl)quinoline-2-carboxaldehyde (FQ). However, since a complicated pretreatment such as an induction sequence of a labeling agent is required, it is not preferred as a simple separation method.

As described above, it has been known that the capillary electrophoresis has an excellent separation capacity but its detection hardly reaches to an ultramicro analysis of the concentration of several ppm or less, for example, 10⁻⁹ M or ppb. For that reason, development in the labeling agent in regard to a novel biological body related substance is expected. As one of the labeling agents, the labeling agent using a rare earth fluorescent complex has excellent properties as the labeling agent such as (1) a strong fluorescence intensity, (2) a long fluorescence lifetime, (3) a stability in solution, and the like.

An attempt to analyze a biosubstance by labeling the biosubstance with a fluorescent metal complex which is generating fluorescence was first made by labeling europiumchelate to an antibody in 1975 (for example, refer to Non-Patent Document 5). After that, accompanying with researches and developments of europium complex which satisfies conditions necessary for detecting the biosubstance such as the long fluorescence lifetime, the strong fluorescence intensity, the stability in solution, and the like, the labeling method called as LKB system was developed by Wallac Inc. (USA). In this method, it is possible to label protein but there are weak points in that the labeled europium itself does not have fluorescence and is significantly affected by an unreacted europium in solution, many reaction routes exist at the time of labeling, a solid phasing is not possible, etc., and thus the method could not be used as it is. Afterward, in order to solve such weak points, a fluorescent europium complex, 4,7-bis(chlorosulfophenyl)-1,10-phenanthroline-2,9-dicarboxylic acid (BCPDA) was synthesized and a method called as CyberFluor system was developed.

There is an example in which a time-resolved immunoassay is carried out by the use of the above method (for example, refer to Non-Patent Document 6). However, there was a weak point that the fluorescence intensity is lower than that of LKB, and thus high sensitive measurement could not be achieved. Many metal complexes were synthesized to make up for such a weak point (for example, with reference to Non-Patent Documents 7 and 8). However, there exist weak points such as weak fluorescence intensity, a weak bonding with biological molecules, or the like and thus synthesis of the labeling agent which allows high sensitive detection could not be performed.

Recently, there has been used the labeling agent for high sensitive analysis of a biochemical substance, particularly for analysis of protein by immunoassay by using a newly synthesized rare earth fluorescent complex (for example, refer to Non-Patent Documents 9 and 10). Specifically, there are examples in which a hybridization assay or an immunoassay using the two different labeling agents is performed. To describe in detail, the hybridization assay is widely used for quantitative detection of DNA or RNA on the basis of the fact that nucleic acid forms double strand with the complementary sequence thereof. In addition, in the documents, B/F separation is not required since an energy transition of the fluorescence is used. In the analysis of protein or antigen, there is an example in that α-fetoprotein (AFP) and Carcioembryonic Antigen (CEA) in human serum are simultaneously analyzed with high sensitivity by using a complex of europium and samarium and thus the utility has been verified. In such methods, by using the rare earth fluorescent complex, the detection limit has been obtained 100 times higher than organic pigments used in the past.

There has been reported an example in which the labeling agent providing such high sensitivity is used and the biological molecules are separated by the separation method used in the past, that is, the high performance liquid chromatography (for example, refer to Non-Patent Document 11). In addition, there is an attempt to separate amino acid by the use of the high performance liquid chromatography by labeling amino acid, but the separation is not perfect. For that reason, the separation analysis by the use of the capillary electrophoresis method which has the number of theoretical plates more than the high performance liquid chromatography is expected. Up to now, there was provided a method of detecting fluorescence by introducing the metal complex after separating the sample (for example, refer to Non-Patent Document 12). In this method, since a system of a detection becomes complicated and it is difficult to completely regulate the binding of metal with a ligand, the method is not suitable for a quantitative analysis. That is, in the state of metal, the rare earth metal constituting the complex often becomes unstable in the analysis under the high voltage and thus there was a case where the fluorescence may not be generated or where the fluorescence intensity may be weakened.

Moreover, since a metal ion constituting the metal complex is strongly attached to the inner wall of the capillary, the separation capacity may be deteriorated. Therefore, in the above mentioned document, the fluorescence is detected by introducing the complex after separating metal. In this case, there are weak points in that it is not suitable for the quantitative analysis because weak fluorescence of the hydrated metal ions such as terbium and the like are hardly avoided and the reaction is not completely regulated. As mentioned above, in spite of the high sensitivity of the labeling agent, the high sensitive analysis by combining with the electrophoresis method has not been provided yet.

In addition, the inventors has proposed a method of analysis using the rare earth metal complex containing europium (Eu) and 4,4'-bis(1",1",1",2",2",3",3"-heptafluoro-4",6"-hexanedione-6"-yl)-chlorosulfo-o -terphenyl (BHHCT) (for example, refer to Patent Document 1). However, the rare earth fluorescent complex proposed in the document is not sufficient because the metal is easily freed from the ligand and the fluorescence is decreased and thus the better one has been required.
Non-Patent Document 1: J. Chromatography, Issue 558, 1991, page 280
Non-Patent Document 2: Science, Vol. 242, pages 562 to 564
Non-Patent Document 3: J. Chromatography, Vol. 545, page 350 (1991)
Non-Patent Document 4: Journal of Chromatography A., Vol. 894, pages 291 to 296 (2000)
Non-Patent Document 5: Tutorials of Cytology, page 313 (1976)
Non-Patent Document 6: Analytical Chemistry, Vol. 60, pages 1,069 to 1,074 (1988)
Non-Patent Document 7: Journal of the American Chemical Society, Vol. 115, pages 11,032 to 11,040 (1993)
Non-Patent Document 8: Journal Immunological Method, Vol. 179, pages 233 to 240 (1995)
Non-Patent Document 9: Protein, Nucleic Acid, and Enzyme, Vol. 48, Issue 11, pages 1,550 to 1,558 (2003)
- Non-Patent Document 10: Materials Integration, Vol. 17, Issue 3, pages 45 to 49 (2003)
Non-Patent Document 11: Chromatography Journal of Separation Sciences, Vol.23, Issue 2, pages 73 to 78 (2002)
Non-Patent Document 12: HRC-Journal of high Resolution Chromatography, Vol. 20, pages 638 to 642 (1997)
Patent Document 1: JP-A-2001-356128

### Disclosure of the Invention

In view of the above-mentioned background, there is provided the use of a rare earth fluorescent complex DTBTA-Eu³⁺ to label a sample in a method of detecting and analysing said sample by electrophoresis.

Preferably, the electrophoresis method is capillary electrophoresis, capillary zone electrophoresis, capillary isoelectric focusing electrophoresis, capillary isotachophoresis, capillary SDS electrophoresis, micellar electrokinetic chromatography, capillary gel electrophoresis, or SDS capillary gel electrophoresis is provided. Preferably , the electrophoresis method is a slab gel electrophoresis, a disc gel electrophoresis, a membrane electrophoresis, a filter paper electrophoresis, a SDS-PAGE, a native-PAGE, an agarose gel electrophoresis, an isoelectric focusing electrophoresis (electrofocusing), or an immunoelectrophoresis. In the use according to a fourth aspect of the invention, a blotting operation is used in combination therewith.

Preferably , the sample is protein, nucleic acid, carbohydrate, or lipid. In the use according to a sixth aspect of the invention, the sample is amino acid.

Preferably , the detection method by the electrophoresis method is a time-resolved detection method.

### Brief Description of the Drawings

Fig. 1 is an observation result of a electrophoresis image of labeled protein in Example 1.
Fig. 2 is a plotted graph for which a migration degree of a band from the electrophoresis image in Example 1 is measured and plotted against a log of molecular weight.
Fig. 3 is an analysis result of DTBTA-Eu³⁺-labeled protein by capillary gel electrophoresis in Example 2.
Fig. 4 is a plotted graph of correlation between a migration time in capillary electrophoresis and each of the molecular weight with respect to a position of peaks in the analysis result of DTBTA-Eu³⁺-labeled protein by capillary gel electrophoresis in Example 2.
Fig. 5 is a high sensitive detection result of DTBTA-Eu³⁺-labeled streptavidin by capillary gel electrophoresis in Example 3.
Fig. 6 is a plotted graph of correlation of the concentrations with respect to a peak intensity in the high sensitive detection result of DTBTA-Eu³⁺-labeled streptavidin by capillary gel electrophoresis in Example 3.
Fig. 7 is a result of the separation analysis of DTBTA-Eu³⁺-labeled protein within the much higher electric field by capillary gel electrophoresis in Example 4.
Fig. 8 is a result of the separation analysis of IgG protein by SDS capillary gel electrophoresis in Example 5.
Fig. 9 is a graph in which the results of the separation analysis of IgG protein by SDS capillary gel electrophoresis in Example 5 are subjected to a regression analysis with the use of a calibration curve obtained in Example 2.
Fig. 10 is an analysis result of protein in Example 2, which is labeled with DTBTA-Eu³⁺ by pulsed-field time-resolved capillary electrophoresis in Example 6.
Fig. 11 is a result of the separation analysis of IgG protein by native capillary gel electrophoresis in Example 7. (a) is a result of native capillary gel electrophoresis of only the antibody labeled with DTBTA-Eu³⁺ and the peak 1 is the peak. (b) is a result of the separation analysis of the antigen-antibody complex by capillary gel electrophoresis and the peak 1 is the peak of the antibody labeled with DTBTA-Eu³⁺ and the peak 2 is the peak of the antigen-antibody complex of the antibody labeled with DTBTA-Eu³⁺ and the unlabeled antigen in the fetal bovine serum.
Fig. 12 is a linear regression graph drawn by the use of the calibration curve which is obtained by the correlation between the concentration of the antigen in the fetal bovine serum obtained in Example 7 and the peak intensity due to the antigen-antibody obtained by native capillary gel electrophoresis.

### Best Mode for Carrying out the Invention

The present invention has the above-mentioned features and the best mode for carrying out the invention will be described below.

The invention provides the use of a rare earth fluorescent complex DTBTA-Eu³⁺ as a labeling agent, to label a sample in a method of detecting and analysing acid sample by electrophoresis.

Here, DTBTA which is a ligand constituting the rare earth fluorescent complex represents 2,2',2",2'"-{[(4,6-dichloro-1,3,5-triazin-2-yl)amino-biphenyl-4-yl]-2,2',6',2"-terpyridine-6,6"-diyl}bis(methylenenitrilo) tetraacetic acid and the rare earth fluorescent complex DTBTA-Eu³⁺ is represented by a following formula.

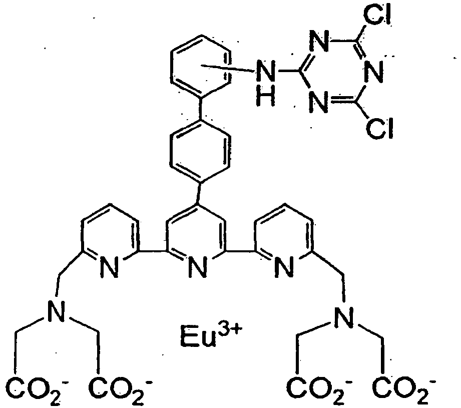

The rare earth fluorescent complex DTBTA-Eu³⁺ is, for example, produced by a method proposed by the present inventors (International Publication No.:
W02003/076938).

As for the electrophoresis method, for example, a capillary electrophoresis method which is performed in the capillary of 100 µm or below is preferred. For using the capillary, even when extremely high electric field (100 to 500 Vcm⁻¹) is applied, the flowing current is small. Therefore, it is possible to suppress the generation of the Joule heat, which has been a big problem in case of performing electrophoresis, to the minimum. Moreover, since the capillary has a large ratio of surface area relative to the inner volume, it is possible to effectively diffuse the generated Joule heat and to obtain high separation efficiency within a short time of analysis. In addition to the capillary electrophoresis method, there may be employed electrophoresis methods such as capillary zone electrophoresis using the hollow capillary, capillary isoelectric focusing electrophoresis, capillary isotachophoresis, capillary SDS electrophoresis, micellar electrokinetic chromatography, capillary gel electrophoresis, or SDS capillary gel electrophoresis.

As for the method of labeling in the invention, a slab gel electrophoresis, a disc gel electrophoresis, a membrane electrophoresis, a filter paper electrophoresis, a SDS-PAGE, a native-PAGE, an agarose gel electrophoresis, an isoelectric focusing electrophoresis (electrofocusing), or an immunoelectrophoresis may be employed. As for the membrane electrophoresis, a cellulose acetate membrane electrophoresis and the like may be used. In addition, a blotting operation may be used in combination therewith.

Among these, in particular, the capillary electrophoresis is preferred because it is excellent in respect of the rapid analysis and dispersion of the Joule heat as compared with the slave gel electrophoresis method.

In the method of labeling , in order to label the sample used in the electrophoresis with the rare earth fluorescent complex, it may be carried out by contacting an object to be examined which has a possibility of containing the electrophoresis sample and the rare earth fluorescent complex. For example, each of the rare earth fluorescent complex DTBTA-Eu³⁺ and the sample is dissolved in a carbonate buffer solution of pH 9.0 to 9.5 and both are blended and reacted, thereby labeling the electrophoresis sample.

As for the samples, protein, nucleic acid, carbohydrate, lipid, and amino acid are preferred. For amino acid, the electrophoresis method such as capillary electrophoresis, capillary zone electrophoresis, capillary isoelectric focusing electrophoresis, capillary isotachophoresis, capillary SDS electrophoresis, micelle conductive electrophoresis, capillary gel electrophoresis, or SDS capillary gel electrophoresis is preferably used.

Further, in the invention, there is provided a method of analyzing and detecting which allows high sensitive analysis of the sample labeled with the rare earth fluorescent complex DTBTA-Eu³⁺ described above by providing an electrophoresis condition for the separation analysis suitable for the labeling agent.

As for such electrophoresis method, as mentioned above, for example, the capillary electrophoresis performed in the capillary of 100 µm or below is preferred. For using the capillary, even when extremely high electric field (100 to 500 Vcm⁻¹) is applied, the flowing current is small. Therefore, it is possible to suppress the generation of Joule heat, which has been a big problem in case of performing electrophoresis, to the minimum.

Moreover, since the capillary has a large ratio of surface area relative to the inner volume, it is possible to effectively diffuse the generated Joule heat and to obtain the high separation efficiency within a short time of analysis. In addition to the capillary electrophoresis, there may be employed electrophoresis such as capillary zone electrophoresis using the hollow capillary, capillary isoelectric focusing electrophoresis, capillary isotachophoresis, capillary SDS electrophoresis, micellar electrokinetic chromatography, capillary gel electrophoresis, or SDS capillary gel electrophoresis.

As for the electrophoresis method in the invention, the slab gel electrophoresis, the disc gel electrophoresis, the membrane electrophoresis, the filter paper electrophoresis, the SDS-PAGE, the native-PAGE, the agarose gel electrophoresis the isoelectric focusing electrophoresis (electrofocusing), or the immunoelectrophoresis may be employed. As for the membrane electrophoresis, the cellulose acetate membrane electrophoresis method and the like are considered. In addition, the blotting operation may be used in combination therewith.

Among these, in particular, the capillary electrophoresis is preferred because the capillary electrophoresis is excellent in respect of the rapid analysis and dispersion of the Joule heat as compared with the slave gel electrophoresis method.

As for the samples, protein, nucleic acid, carbohydrate, lipid, and amino acid are preferred. For amino acid, the electrophoresis method such as capillary electrophoresis, capillary zone electrophoresis, capillary isoelectric focusing electrophoresis, capillary isotachophoresis, capillary SDS electrophoresis, micellar electrokinetic chromatography, capillary gel electrophoresis, or SDS capillary gel electrophoresis is preferably used.

The method of analyzing and detecting enables the separation analysis to be performed by capillary electrophoresis in the electric fields, for example, under the several kV of voltages by labeling protein with the metal complex DTBTA, in which the fluorescence is not disappeared but stable. In addition, the complex can be generally used in the general plate gel electrophoresis as mentioned above.

The inner wall of the capillary is usually negatively charged. However, the charge is eliminated by treating the inner wall so that an interaction between the metal constituting the complex and the inner wall is alleviated.

Particularly, in respect of performing a high sensitive analysis, the detection method by absorption or fluorescence used in the past has the detection limit of no more than mM degrees due to the absorption by a polymer and a buffer solution that are filled the inner part or the background fluorescence. For that reason, in order to make use of the excellent characteristic of the labeling agent, by additionally using a time-resolved method in capillary electrophoresis, a signal from the labeling agent can be more effectively measured. By doing so, for example, the background signal from molecular sieve materials such as a filled gel, a polymer, or the like is reduced and the measurement avoiding the fluorescence from the buffer solution or an adulteration is possible. That is, since the lifetime of the fluorescence from the polymer or the buffer solution is 10 nano seconds or less, the detection sensitivity can be increased by measuring the fluorescence after excluding the fluorescence from the polymer or the buffer solution. Here, the molecular sieve materials mean a filler to give a sieve effect to the migration of the sample such as a gel, a polymer, or the like filled the inner part of the capillary.

According to the method of analyzing and detecting , by making use of the high quantum efficiency that the metal complex generally has, the high sensitive analysis in which allows the detection limit to be increased up to approximately 100 times becomes possible. This advantage is generated also by the big Stokes shift of the metal complex.

Hereinafter, the invention will be further described with reference to Examples in detail. Of course, the invention is not limited to the following Examples.

### EXAMPLES

### <EXAMPLE 1>

### <Preparation of Protein Labeled with DTBTA-Eu-3⁺>

Protein used in the invention was dissolved in 100 mM of a carbonate buffer solution (pH 9.1) such to give 2 mg/ml. DTBTA-Eu³⁺ was dissolved in 100 mM of carbonate buffer solution (pH 9.1) such to give 1 mg/ml and mixed with the above protein solution to give a volume ratio of 1 : 1, and then reacted for 3 hours at a room temperature. After the reaction, unreacted DTBTA-Eu³⁺ was removed by a gel filtration by the use of a Sephadex^{™} G-25 column and the labeled protein fraction was prepared.

### <Analysis of Protein Labeled with DTBTA-Eu³⁺ by Slave Gel>

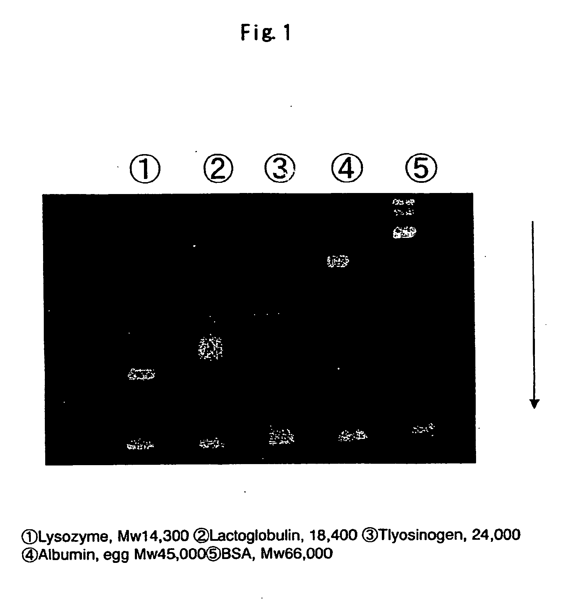

SDS-PAGE of protein labeled with DTBTA-Eu³⁺ was carried out by using Laemmli system. As for the separation gel, 12 to 17.5% of a gradient gel was used. After the migration, UV light was irradiated to the gel by UV trans-illuminator and the electrophoresis image of the labeled protein was observed. A band having the Eu³⁺ complex's own red fluorescence was identified. The results are shown in Fig. 1.

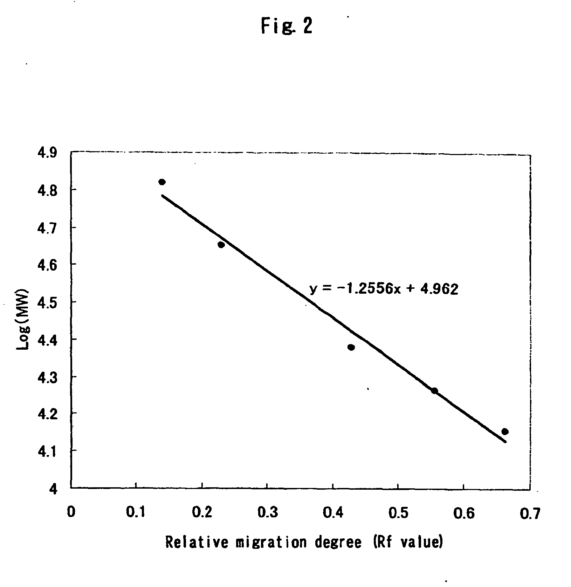

The migration degree of the band was measured from the electrophoresis image and was plotted against the log of the molecular weight, and then a satisfactory linear relation was obtained. The results are shown in Fig. 2.

### <EXAMPLE 2>

### <Analysis of DTBTA-Eu³⁺ -Labeled Protein by Capillary Gel Electrophoresis>

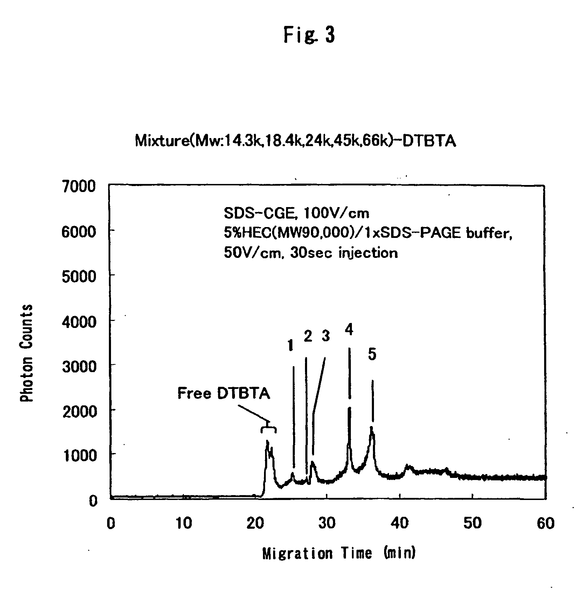

SDS capillary gel electrophoresis was carried out at 4,000 V of applied voltage (100 V/cm of electric field intensity) for the sample of protein labeled with DTBTA-Eu³⁺ prepared in Example 1. The separation was carried out by using 5 % (W/W) of a replaceable hydroxyethyl cellulose buffer solution. 5 % (W/W) of the hydroxyethyl cellulose buffer solution was prepared by the use of a commercially available hydroxyethyl cellulose buffer solution, in this case, was prepared by the use of pH 8.3 of Tris/Glycine/SDS, which is generally used for the separation analysis of protein, such to give 5% in weight percentage. The detection was carried out by the use of the time-resolved capillary electrophoresis system under the conditions of 350 nm of excitation, 610 nm of detection, and 100 µsec of retarded fluorescence detection. The results are shown in Fig. 3.

In Fig. 3, each band assigns 1: Lysozyme, 8 nM, MW= 14,300, 2: b-Lactoglobulin, 6.6 nM, MW=18,300, 3: Tlypsinogen, 5.4 nM, MW=24,000, 4: Albumin, egg, 2.8 nM, MW=45,000, and 5: BSA (Bovine Serum Albumin), 2 nM, MW=66,000.

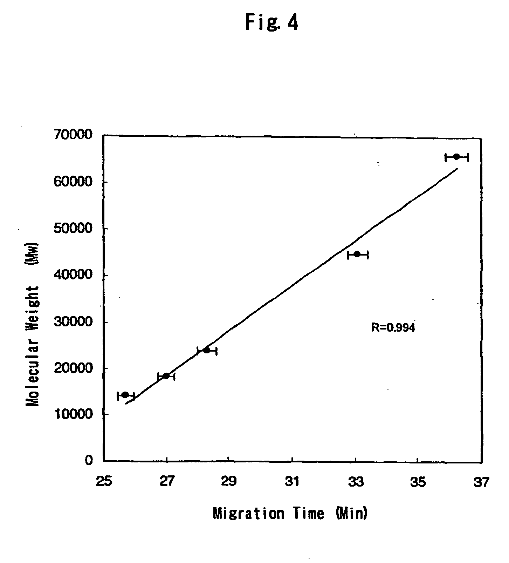

For the position of the peak, the migration degree was calculated and its correlation with each molecular weight was investigated, and then a satisfactory linear relation was obtained. The results are shown in Fig. 4.

### <EXAMPLE 3>

### <High Sensitive Detection of DTBTA-Eu³⁺-Labeled Streptavidin by Capillary Gel Electrophoresis>

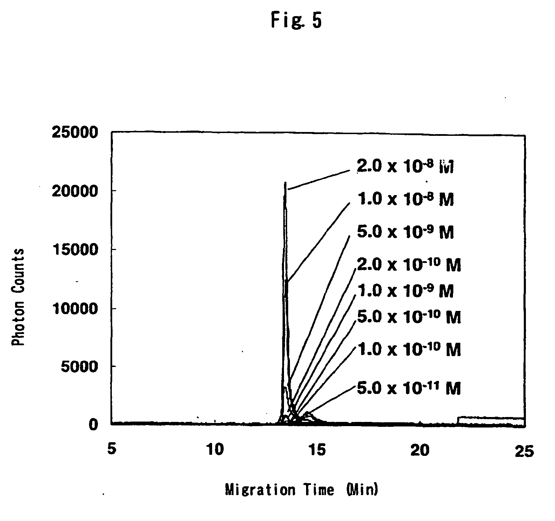

SDS capillary gel electrophoresis was carried out at 4,000 V of applied voltage (100 V/cm of electric field intensity) for the sample of streptavidin (S-Avidin) labeled with DTBTA-Eu³⁺. The separation was carried out by using 1 % (W/W) of the replaceable hydroxyethyl cellulose buffer solution. 5% (W/W) of the hydroxyethyl cellulose buffer solution was prepared by the use of the commercially available hydroxyethyl cellulose buffer solution such to give 5% in weight percentage, in this case, was prepared by the use of pH 8.3 of Tris/Glycine/SDS, which is generally used for the separation analysis of protein. The detection was carried out by the use of the time-resolved capillary electrophoresis system under the conditions of 350 nm of excitation, 610 nm of detection, and 100 µsec of retarded fluorescence detection. The results are shown in Fig. 5.

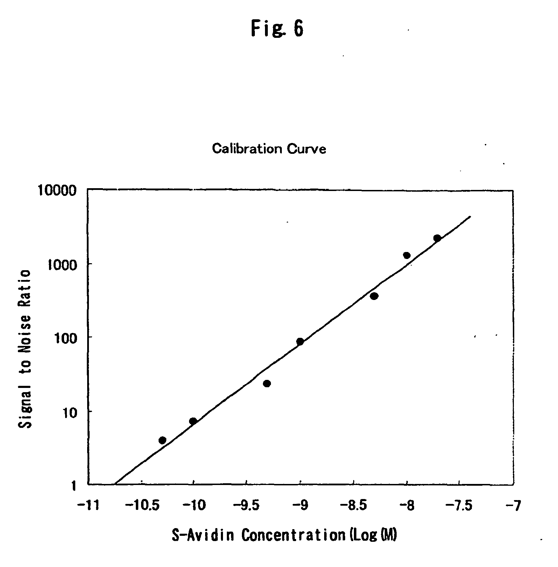

For the intensity of the peak, the correlation with the concentrations was obtained as a satisfactory straight line. The results are shown in Fig. 6. The detection limit was 5×10⁻¹¹ M(S/N ratio=3.9).

### <EXAMPLE 4>

### <Separation Analysis of DTBTA-Eu³⁺-Labeled Protein in High Electric Field by Capillary Gel Electrophoresis>

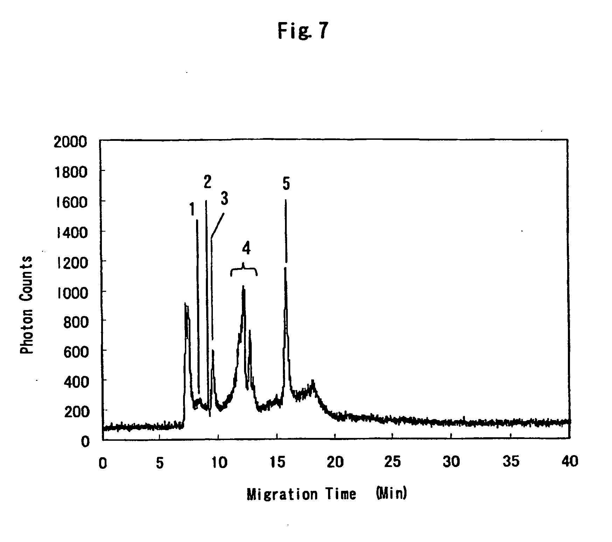

SDS capillary gel electrophoresis was carried out at 10,000 V of applied voltage (250 V/cm of electric field intensity) for the sample of protein labeled with DTBTA-Eu³⁺ which was prepared in Example 1. The concentration of the molecular sieve and the detection conditions are the same as Example 2. The results are shown in Fig. 7.

In Fig. 7, each band assigns 1: Lysozyme, MW=14,300, 2: b-Lactoglobulin, MW=18,300, 3: Tlypsinogen, MW=24,000, 4: Albumin, egg, MW=45,000, and 5: BSA (Bovine Serum Albumin), MW=66,000.

### <EXAMPLE 5>

### <SDS Capillary Gel Electrophoresis of IgG Protein>

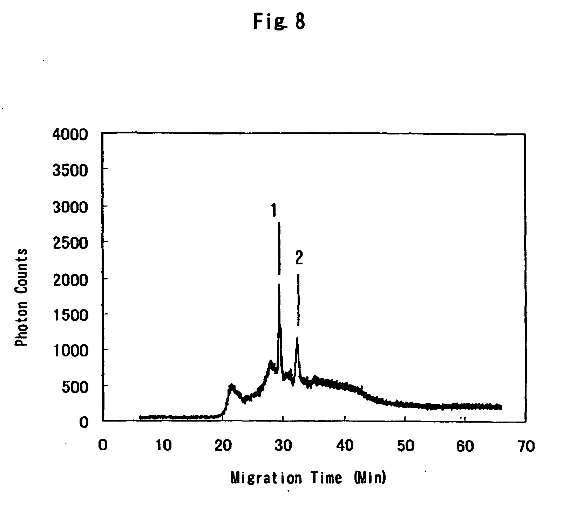

IgG labeled with DTBTA-Eu³⁺ was treated with a reducing agent and subjected to the separation analysis under the same condition as Example 2, and then the bands of a long chain and a light chain were identified. The results are shown in Fig. 8.

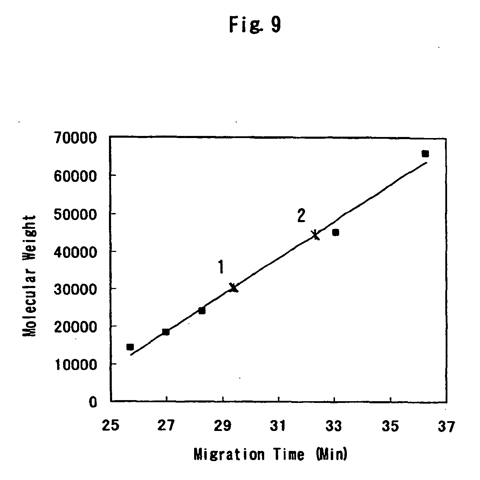

When the results are subjected to a regression analysis with the use of the calibration curve obtained in Example 2 and the molecular weight of the long chain was approximately 44,000 and the molecular weight of the light chain was approximately 30,000. The results are shown in Fig. 9.

In Fig. 9, each band assigns 1: Light chain and 2: Long chain.

### <EXAMPLE 6>

### <Pulsed-Field Time-Resolved Capillary Electrophoresis>

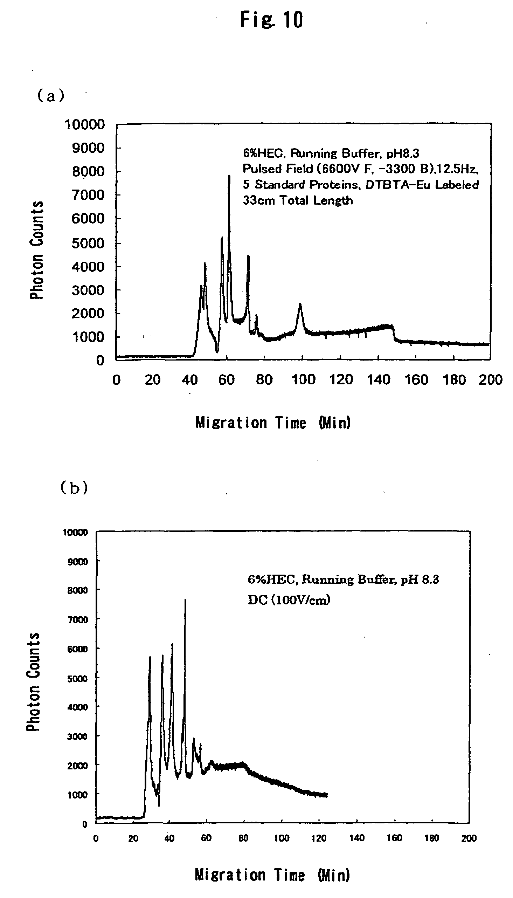

The sample of protein labeled with DTBTA-Eu³⁺ prepared in Example 1 was subjected to the separation analysis by a rectangular wave. By applying voltages of 6,600 V in a forward direction and 3,300 V in a reverse direction with respect to the migration direction at the rate of 12.5 Hz, the separation analysis was carried out by capillary gel electrophoresis. The separation was carried out by using 6 % (W/W) of the replaceable hydroxyethyl cellulose buffer solution. The detection was carried out by the use of the time-resolved capillary electrophoresis system under the conditions of 350 nm of excitation, 610 nm of detection, and 100 µsec of retarded fluorescence detection. 6% (W/W) of the hydroxyethyl cellulose buffer solution was prepared by the use of the commercially available, hydroxyethyl cellulose buffer solution such to give 6 % in weight percentage, in this case, was prepared by the use of pH 8.3 of Tris/Glycine/SDS which is generally used for the separation analysis of protein. The results are shown in Fig. 10(a) and (b). (a) represents a result of pulsed-field time-resolved capillary electrophoresis and (b) represents a result from direct current wave (3,300 VDC) which was carried out for a control experiment.

In each peak in Fig. 10(a) and (b), it is confirmed that the peak 1 which is the peak of BSA is well separated in pulsed-field time-resolved capillary electrophoresis.

From the above results, when separating the protein molecule having large molecular weight, the separation is further processed by performing the separation analysis by the rectangular wave, that is, pulsed-field time-resolved capillary electrophoresis.

### <EXAMPLE 7>

### <Immunoassay and Native Time-Resolved Capillary Electrophoresis>

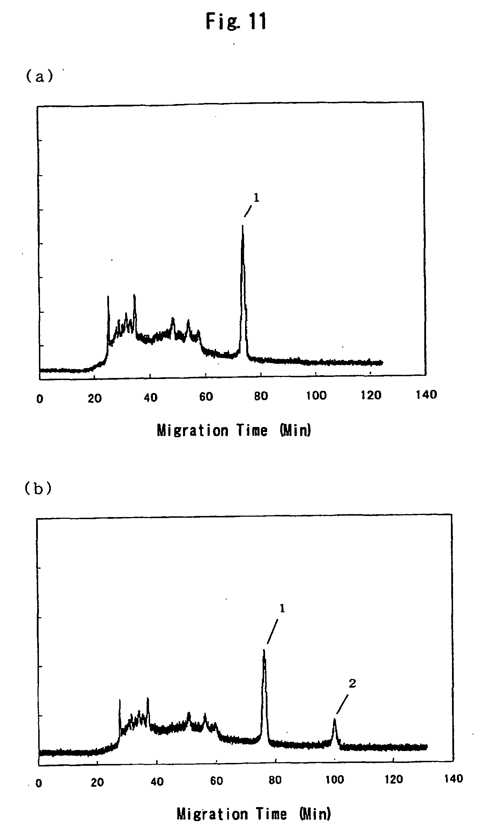

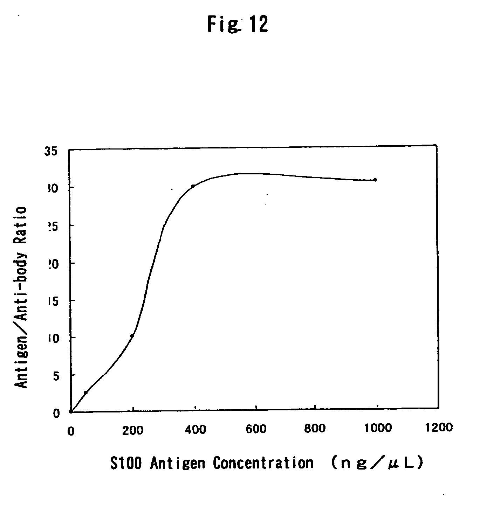

Immunoassay was carried out by using S100 protein as an antigen and the analysis was carried out in a fetal bovine serum by time-resolved capillary gel electrophoresis. S100 antibody was labeled with DTBTA-Eu³⁺ and the quantity of S100 protein (antigen) in the fetal bovine serum was determined. A method of labeling the S100 antibody with DTBTA-Eu³⁺ was carried out in the same manner as shown in Example 1. For the separation analysis, the analysis was carried out by native capillary electrophoresis which performs the separation analysis of the antigen-antibody as it is without using SDS and the reducing agent. The separation was carried out by using 5% (W/W) of the replaceable hydroxyethyl cellulose buffer solution. The detection was carried out by the use of the time-resolved capillary electrophoresis system under the conditions of 350 nm of excitation, 610 nm of detection, and 100 µsec of retarded fluorescence detection. In this experiment, the length of the capillary was 33 cm and the applied voltage was 3,300 V (100 V/cm). Other conditions such as buffer solution are the same as Example 2. The antigen-antibody reaction was carried out by mixing the labeled S100 antibody with S100 protein (antigen) added in the fetal bovine serum and reacted for 1 hour. The results are shown in Fig. 11(a) and (b) and Fig. 12.

Fig. 11 (a) is a result of native capillary gel electrophoresis of only the antibody labeled with DTBTA-Eu³⁺ and the peak 1 is the peak of the labeled antibody. (b) is a result that the antigen-antibody complex is prepared in the fetal bovine serum and is separated and analyzed by capillary gel electrophoresis. The peak 1 is the peak of the antibody labeled with DTBTA-Eu³⁺ and the peak 2 is the peak of the antigen-antibody complex of the antibody labeled with DTBTA-Eu³⁺ and the unlabeled antigen of the fetal bovine serum. In addition, measurement was performed after changing the concentration of S100 protein to be added in the fetal bovine serum. The resultant calibration curve is Fig. 12. It has been found that the antigen can be measured by the native method by the use of the time-resolved capillary electrophoresis method.

## Claims

1. The use of a rare earth fluorescent complex DTBTA-Eu³⁺ to label a sample in a method of detecting and analysing said sample by electrophoresis.

2. The use according to Claim 1, wherein the electrophoresis method is one of capillary electrophoresis, capillary zone electrophoresis, capillary isoelectric focusing electrophoresis, capillary isotachophoresis, capillary SDS electrophoresis, micellar electrokinetic chromatography, capillary gel electrophoresis, and SDS capillary gel electrophoresis.

3. The use according to Claim 1, wherein the electrophoresis method is one of a slab gel electrophoresis, a disc gel electrophoresis, a membrane electrophoresis, a filter paper electrophoresis, a SDS-PAGE, a native-PAGE, an agarose gel electrophoresis, an isoelectric focusing electrophoresis (electrofocusing), and an immunoelectrophoresis.

4. The use according to Claim 3, wherein a blotting operation is used in combination therewith.

5. The use according to any one of Claims 1 to 4, wherein the sample is one of protein, nucleic acid, carbohydrate, and lipid.

6. The use according to Claim 2, wherein the sample is amino acid.

7. The use according to Claim 2, wherein the detection method by the electrophoresis method is a time-resolved detection method.

## Patentansprüche

1. Verwendung eines Seltenerd-Fluoreszenzkomplexes DTBTA-Eu³⁺, um eine Probe in einem Verfahren zum Detektieren und Analysieren der Probe durch Elektrophorese zu kennzeichnen.

2. Verwendung nach Anspruch 1, wobei das Elektrophoreseverfahren eines der folgenden Verfahren ist: Kapillar-Elektrophorese, Kapillarzonen-Elektrophorese, Kapillar-Elektrophorese mit isoelektrischer Fokussierung, Kapillar-Isotachophorese, Kapillar-SDS-Elektrophorese, micellare elektrokinetische Chromatographie, Kapillar-Gel-Elektrophorese und SDS-Kapillar-Gel-Elektrophorese.

3. Verwendung nach Anspruch 1, wobei das Elektrophoreseverfahren eines der folgenden Verfahren ist: Platten-Gel-Elektrophorese, Scheiben-Gel-Elektrophorese, Membran-Elektrophorese, Filterpapier-Elektrophorese, SDS-PAGE, native PAGE, Agarose-Gel-Elektrophorese, Elektrophorese mit isoelektrischer Fokussierung (Elektrofokussierung) und Immuno-Elektrophorese.

4. Verfahren nach Anspruch 3, wobei in Kombination hiermit eine Blotting-Operation verwendet wird.

5. Verwendung nach einem der Ansprüche 1 bis 4, wobei die Probe ein Protein oder eine Nukleinsäure oder ein Kohlenstoffhydrat oder ein Lipid ist.

6. Verwendung nach Anspruch 2, wobei die Probe eine Aminosäure ist.

7. Verwendung nach Anspruch 2, wobei das Detektionsverfahren durch das Elektrophoreseverfahren ein zeitlich aufgelöstes Detektionsverfahren ist.

## Revendications

1. Utilisation d'un complexe fluorescent de terres rares DTBTA-Eu³⁺ pour marquer un échantillon dans un procédé de détection et d'analyse dudit échantillon par électrophorèse.

2. Utilisation selon la revendication 1, dans laquelle le procédé d'électrophorèse est l'un parmi une électrophorèse capillaire, une électrophorèse capillaire de zone, une électrophorèse capillaire de focalisation isoélectrique, une isotachophorèse capillaire, une électrophorèse SDS capillaire, une chromatographie électrocinétique micellaire, une électrophorèse sur gel capillaire, et une électrophorèse SDS sur gel capillaire.

3. Utilisation selon la revendication 1, dans laquelle le procédé d'électrophorèse est l'un parmi une électrophorèse sur gel en tranches, une électrophorèse sur gel de disque, une électrophorèse de type membrane, une électrophorèse sur papier filtre, un SDS-PAGE, un PAGE natif, une électrophorèse sur gel d'agarose, une électrophorèse de focalisation isoélectrique (électrofocalisation), et une immunoélectro-phorèse.

4. Utilisation selon la revendication 3, dans laquelle une opération de buvardage est utilisée en combinaison avec celle-ci.

5. Utilisation selon l'une quelconque des revendications 1 à 4, dans laquelle l'échantillon est l'un parmi une protéine, un acide nucléique, un glucide et un lipide.

6. Utilisation selon la revendication 2, dans laquelle l'échantillon est un acide aminé.

7. Utilisation selon la revendication 2, dans laquelle le procédé de détection par le procédé d'électrophorèse est un procédé de détection à résolution dans le temps.
